# EUROPEAN PATENT SPECIFICATION

(11) **EP 2 588 084 B1**
(45) Date of publication and mention of the grant of the patent: **22.11.2023**
(21) Application number: 11728632.8
(22) Date of filing: 30.06.2011
(51) Int. Cl.: A61K 9/19, A61K 31/7048, A61K 47/10, A61P 31/04, A61P 33/02

(54) **Antibiotic compositions comprising tulathromycin**
Antibiotische Zusammensetzungen enthaltend Tulathromycin
Compositions antibiotiques comprenant tulathromycine

(30) Priority: 01.07.2010 EP 10168166
(43) Date of publication of application: 08.05.2013
(73) Proprietor: Elanco Tiergesundheit AG, 4058 Basel (CH)
(72) Inventor: HOTTER, Andreas, A-6250 Kundl (AT); LUDESCHER, Johannes, A-6250 Kundl (AT); GARCIA, Rafael, E-08400 Granollers (ES); MARTORELL, Oriol, E-08400 Granollers (ES); CODONY, Albert, E-08400 Granollers (ES)
(74) Representative: Greaves Brewster LLP
(86) International application number: PCT/EP2011/060986
(87) International publication number: WO 2012/001089

(56) References cited:
- WO-A1-02/088158
- WO-A1-2006/115494
- WO-A2-03/011266
- US-A1- 2003 013 860

## Description

The present invention relates to a pharmaceutical or veterinary kit for administering a tulathromycin and to a method for the preparation of a composition comprising a tulathromycin.

Draxxin^{®} (tulathromycin) is a semi synthetic microcline antibiotic indicated e.g. for the treatment of swine respiratory disease, for the treatment of bovine respiratory disease and for the control of respiratory disease in cattle at high risk to develop bovine respiratory disease as well as for the treatment of infectious bovine keratoconjuctivis.

Draxxin is available as injectable solution consisting of an equilibrated mixture of two chemical isomers,
a) the compound of formula I, designated as Tulathromycin A with the chemical name (2R,3S,4R,5R,8R,10R,11R,12S,13S,14R)-13-(2,6-dideoxy-3-C-methyl-3-O-methyl-4-C-((propylamino)-methyl)-α-L-ribo-hexopyranosyl)oxy-2-ethyl-3,4,10-trihydroxy-3,5,8,10,12,14-hexamethyl-11-((3,4,6-trideoxy-3-(dimethylamino)-β-D-xylo-hexopyranosyl)oxy)-1-oxa-6-azacyclopentadecan-15-one. 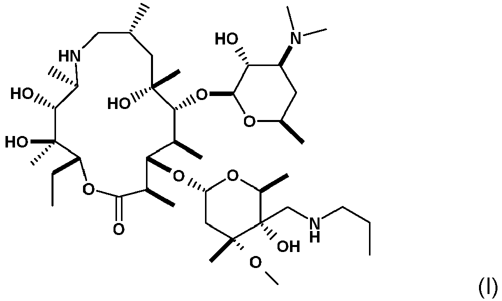
b) The compound of formula II, designated as Tulathromycin B with the chemical name (3R, 6R8R, 9R, 10S, 11S, 12R)-11-((2,6-dideoxy-3-C-methyl-3-O-methyl-4-C-((propylamino)methyl-a-L-ribo-hexopyranosyl)oxy)2-((1R,2R)-1,2-dihydroxy-1-methylbutyl)-8-hydroxy-3,6,8,10,12-pentamethyl-9-((3,4,6-trideoxy-3-(dimethylamino)-β-D-xylohexopyranosyl)oxy)-1-oxa-4-azacyclotridecan-13-one.

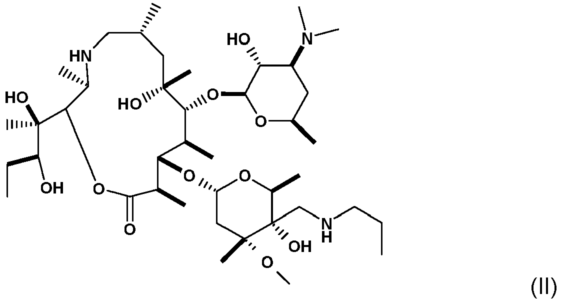

Compound of formula II can be formed from a translactonization reaction of isomer I. Draxxin^{®} is marketed as an injectable solution comprising an equilibrium mixture of about 90% ± 4% of isomer A and about 10% ± 4% of isomer B.

WO 02/088158 discloses processes for preparing C-4" substituted derivatives of 9-deoxo-9a-aza-9a-homoerythromycin A that are useful as antibacterial and antiprotozoal agents in mammals.

Solutions of pharmaceutical ingredients have a limited chemical stability compared to solid forms, as solvents, especially water, slowly degrades the active pharmaceutical ingredient. In addition, in order reach the equilibribium mixture in Draxxin^{®}, critical parameters have to be watched very carefully as pH, temperature and time heavily influence the quality of the equilibrated mixture with respect to impurities. One approach to overcome the problem comprises adding a colsolvent to the formulation in order to improve the stability of the equilibrated mixture in solution, see e.g. US 6,667,393 or US 6,514,945.

However, a solid formulation which is reconstituted with a solvent just before injection, is preferred to a solution. Development of a solid pharmaceutical composition comprising a single Tulathromycin isomer in high purity is therefore desirable.

The present invention relates to pharmaceutical and veterinary kits for administering a tulathromycin that consists of more than 98% of Tulathromycin A and less than 2% of Tulathromycin B to a mammal, comprising a first and a second container, wherein the first container comprises a lyophilized composition of a tulathromycin that consists of more than 98% of Tulathromycin A and less than 2% of Tulathromycin B, in form of an addition salt with citric acid or a mixture of citric acid and hydrochloric acid, and the second container comprises a solvent comprising water and optionally a water-miscible solvent, an antioxidant and/or a preservative. Upon the addition of a solvent or diluent the solid formulation may be transferred to a ready-to-use injectable before injection.

Essentially pure Tulathromycin A means pure with respect to the presence of Tulathromycin B including a high chemical purity of Tulathromycin. Within the present disclosure "essentially pure" means the presence of more than about 97 %, more preferably more than 98%, e.g. 99% of Tulathromycin A and less than about 3%, preferably less than about 2%, e.g. about 1% of Tulathromycin B in said composition.

Suitable acids for forming an addition salt with Tulathromycin A are, for example, acetic acid, benzenesulfonic acid, p-toluenesulfonic acid, citric acid, methanesulfonic acid, hydrochloric acid, D- and L-lactic acid, succinic acid, sulfuric acid, D- and L. tartraric acid, malic acid, malonic acid, fumaric acid, palmitic acid, benzoic acid and phosphoric acid, preferably citric acid or a mixture of citric acid and hydrochloric acid.

The compositions of the invention contain Tulathromycin A in form of an addition salt with citric acid or as addition salt with two different acids selected from the acids as mentioned above, in particular with citric acid and hydrochloric acid. The solid compositions of the invention may contain, in addition, some free acid, preferably selected from the one or more acids used to prepare the addition salt with Tulathromycin A, for example, for adjusting the pH value after reconstitution.

The addition salt of Tulathromycin A is present in the compositions of the present invention, for example, in crystalline form or preferably in amorphous form.

The present invention also relates to a method of preparing a lyophilized composition comprising a tulathromycin that consists of more than 98% of Tulathromycin A and less than 2% of Tulathromycin B, in form of an addition salt with citric acid or a mixture of citric acid and hydrochloric acid, comprising the steps of:
a) dissolving the tulathromycin in water with citric acid or a mixture of citric acid and hydrochloric acid,
b) adjusting the pH to a pH of 4.0 to 8.0, and
c) lyophilizing the mixture from step a) or b).

In general, a solution of substantially pure Tulathromycin is obtained by dissolving Tulathromycin base in water in the presence of one or more acids and lyophilizing the obtained solution, preferably after a sterile filtration step.

Any form of essentially pure Tulathromycin A may be used as starting material. The starting material of Tulathromycdin A may be in crystalline form or amorphous form. In addition, salts of Tulathromycin may be used, for example a hydrochloride, citrate or phosphate. In case addition salts such as a hydrochloride or citrate are used as starting material, additional acids optionally have to be added in order to adjust the pH accordingly. In case of an addition salt which is not pharmaceutically or veterinary acceptable, said salt previously has to be transformed to the required pharmaceutically or veterinarily acceptable addition salt e.g. by extraction of the free base or by other methods before performing the steps of the present invention.

Suitable acids useful for obtaining a solution of substantially pure Tulathromycin A include, for example, the acids as mentioned above. Preferably, two different acids selected from the acids as mentioned above, in particular citric acid and hydrochloric acid, are employed in the method of the invention.

If citric acid is used, the amount thereof present in the mixture is from about 1,0 mol per mol of substantially pure Tulathromycin A to about 4 mol of citric acid per mol of Tulathromycin A, more preferably from about 1,1 to about 3 mols, citric acid per mol of substantially pure Tulathromycin when citric acid is the only acid present.

If a combination of citric acid and hydrochloric acid is used, the amount of citric acid present is from about 0.5 mol per mol of substantially pure Tulathromycin A to about 4 mol of citric acid per mol of Tulathromycin A, more preferably from about 0.6 to about 2 mols, more preferably from about 0.7 mol to 1.2 mol citric acid per mol of substantially pure Tulathromycin.

The amount of hydrochloric acid present if a combination of citric acid and hydrochloric acid is used is from about 0 to about 3 mols per mole of substantially pure Tulathromycin A, more preferably from about 1, 5 to mols of hydrochloride acid to about 3 mols of hydrochloric acid. Additional hydrochloric acid may be present depending on the final matrix/concentration of the ready to use formulation in order to have a pH of about 4.0 to 8.0 , more preferable about 4,5 to 7,5 before administration.

The aqueous solution of Tulathromycin A and the one or more acids, in particular hydrochloric acid and citric acid, is then lyophilized to obtain a solid composition of substantially pure Tulathromycin A as addition salt with the one or more acids, in particular with hydrochloric acid and citric acid.

The compositions of the present invention are very stable, e.g. when stressed at elevated temperature even for a prolonged time; only a small increase in Tulathromycin B and only a moderate increase of impurities is observed in each case.

Surprisingly, upon reconstitution with water only, the solution of substantially pure Tulathromycin A is stable at ambient temperature and no significant increase in impurities can be detected even after a time period of 7 days in absence of any antioxidant or of any cosolvent. Isomerization to Tulathromycin B takes place only at a limited rate, thus the administration of substantially pure Tulathromycin A as defined above is guaranteed over a long period of time exceeding reasonably standard time intervals between dissolution and administration by far.

The present invention relates to a pharmaceutical or veterinary kit for administering substantially pure Tulathromycin A to a mammal, comprising a first and a second container, wherein the first container comprises a composition comprising essentially pure Tulathromycin A in form of an addition salt with one or more acids, and the second container contains a solvent comprising water and optionally one or more water-miscible solvents, one or more antioxidants and/or one or more preservatives.

Concerning the composition of the Tulathromycin A of the first container, the above-given meanings and preferences apply. In addition, the composition comprising the essentially pure Tulathromycin A in form of an addition salt with one or more acids may be prepared as described above.

The solvent of the second container may contain, in addition to water, one or more water-miscible solvents, e.g. for the purpose of retarding a possible byproduct formation upon prolonged standing of the reconstituted solution or any pain related issue upon injection and the like.

Suitable water-miscible solvents include, for example, alcohols, e.g. ethanol or isopropanol, glycols, e.g. diethylene glycol, glycol ethers such as diethylenglycol monomethylether, diethyleneglycol butylether, diethylenglycol dibutylether, diethylenglycol monoethylether, polyethylenglycols such as polyethylene glycol-300 or polyethylene glycol-400 , propylene glycol, glicerine, pyrrolidones, e.g. 2-pyrrolidone or N-methyl 2-pyrrolidone, glycerol formal, dimethyl sulfoxyde, dibutyl sebecate, polyoxyethylene sorbitan esters , e.g. polysorbate 80 or e.g. propylenglycol.

The amount of water-miscible solvent, if present, may vary, for example, from about 10% to 50% by weight of the entire solvent of the second container.

Additionally, an antioxidant may be present in the solvent of the second container. Suitable antioxidants include, for example, sodium bisulfite, sodium sulfite, sodium thiosulfate, sodium formaldehyde sulfoxylate, L-ascorbic acid, acetylcysteine, cysteine, monothioglycerol, thioglycollic acid, thiolactic acid, thiourea, dithioerithrol, dithioerythreitol, gluthadione, ascorbyl palmitate, butylated hydroxyanisol, butylated hydroxytoluene, nordihydroguaiaretic acid, propyl gallatae, α-tocopherol and mixtures thereof.

The amount of antioxidant in the solvent, if present, may vary from about 0.01 to about 1.0 mol per mol of substantially pure Tulathromycin A contained in the first container, and preferably from about 0.2 to 0.6 mol per mole of substantially pure Tulathromycin A.

In one embodiment of the invention, the solvent of the second container of the kit may also contain one or more preservatives selected, for example, from benzalkonium chloride, benzethoinium chloride, benzoic acid, benzylalcohol, methylparaben, ethylparaben, sodium benzoate, propylparaben, butylparaben , phenol or mixtures thereof. The amount of preservative, if present, is preferably from about 0.01 to about 10 mg per ml of the entire solution of the second container.

The solvent of the second container may be prepared by known processes, for example by simple mixing of water and the optional further components such as water-miscible solvent, antioxidant and/or preservative.

The pharmaceutical or veterinary kits may be used in a method for treating a bacterial or protozoal infection in a mammal, characterized, for example, by the steps of
a) providing a solid composition of an essentially pure Tulathromycin A in form of an addition salt with one or more acids as described above including the given preferences,
b) reconstituting the essentially pure Tulathromycin A with a solvent comprising water and optionally a water-miscible solvent, an antioxidant and/or a preservative, wherein each the above-given meanings and preferences apply, and
c) administering to a mammal in need of such treatment a therapeutically effective amount of the solution obtained according to step b).

A suitable injectable solution of Tulathromycin A for treating a bacterial or protozoal infection in a mammal is prepared, for example, by combining the contents of the first and second container of the kit as described above, preferably just before use; preferably, following said reconstitution the solution comprises a final concentration of about 100 mg/ml of substantially pure Tulathromycin A. In addition, the injectable solution comprises a pH of, for example, from about pH 4.0 to about pH 8.0, preferably from pH 5 to 7.5.

### EXAMPLES

HPLC :
Equipment: Liquid Chromatograph Waters 2695/Hewlett-Packard 1100 or similar.
Detection: by UV at 210 nm.
Column: Waters XTerra MS C₁₈, 250 × 4.6 mm, 5 µm or similar.
Mobile phase:
Mobile phase A : Dissolve 1.80 grams of anhydrous sodium phosphate dibasic in 1000.0 mL of water. Adjust to pH 8.0 ± 0.1 with phosphoric acid or NaOH 1N.
Mobile phase B : Mix 750 mL of acetonitrile and 250 mL of methanol.

Temperature: 60 °C
Injection Volume: 50 µL
Flow: 0.9 mL/min
Run Time: 90 min.
Mode: Gradient mode.
Gradient:

| Time (min) | %A | %B |
|---|---|---|
| 0 | 50 | 50 |
| 25 | 45 | 55 |
| 30 | 40 | 60 |
| 80 | 25 | 75 |
| 81 | 50 | 50 |
| 90 | 50 | 50 |

Solutions:
a) Dilution solvent:
   Dissolve 1.73 grams of ammonium phosphate monobasic in water and take to 1000.0 mL with water. Adjust pH to 10.00±0.05 with ammonium hydroxide solution (59 mL of concentrated ammonia to 100.0 mL with water).
   Take 350 mL of this solution and mix with 300 mL of acetonitrile and 350 mL of methanol.
b) Test solution:
   Dissolve 40.0 mg of the substance to be examined in dilution solvent and dilute to 5.0 mL with dilution solvent.

### Example 1

2.14 g of Tulathromycin base are dissolved in 20 mL of water with 0.41 g of anhydrous citric acid and 0.61 g of hydrochloric acid 10% aqueous solution. The pH of the solution is adjusted to 5.5 by addition of hydrochloric acid 10% aqueous solution. The solution is then lyophilized and an amorphous solid is obtained.

### Example 2

0.5 g of the solid composition obtained according to Example 1 are stressed at a temperature of 80°C for 26 hours. Degradation is studied:

| Table 1 | | | |
|---|---|---|---|
| Degradation time at 80°C | Tulathromycin A | Tulathromycin B | Increase of impurity level |
| Starting material | 96.0% | 0.2% | - |
| 9 hours | 95.2% | 0.3% | 0,7 % |
| 26 hours | 93.6% | 0.3% | 2,3% |

### Example 3

0.5 g of the solid composition obtained according to Example 1 are dissolved in 5 ml of water. The solution is left at room temperature for 7 days and degradation is studied:

| Table 2 | | | |
|---|---|---|---|
| Time in solution | Tulathromycin A | Tulathromycin B | Increase of impurity level |
| Lyophilized material | 96.0% | 0.2% | - |
| 90 minutes | 96.0% | 0.2% | 0.0% |
| 6 hours | 95.9% | 0.3% | 0.0% |
| 7 days | 95.7% | 0.4% | 0.1% |

### Example 4

Tulathromycin A, 2,28 g are suspended in water. 1M citric acid are added until total dissolution (1.1 equivalents). The resultant mixture is lyophilized to yield 2.80 g of amorphous product.

No change in isomer ratio and impurities was observed by HPLC.

## Claims

1. Pharmaceutical or veterinary kit for administering a tulathromycin that consists of more than 98% of Tulathromycin A and less than 2% of Tulathromycin B to a mammal, comprising a first and a second container, wherein
the first container comprises a lyophilized composition of a tulathromycin that consists of more than 98% of Tulathromycin A and less than 2% of Tulathromycin B, in form of an addition salt with citric acid or a mixture of citric acid and hydrochloric acid, and
the second container comprises a solvent comprising water and optionally a water-miscible solvent, an antioxidant and/or a preservative.

2. A pharmaceutical or veterinary kit according to claim 1, wherein the solvent of the second container comprises a water-miscible solvent selected from the group consisting of ethanol, isopropanol, diethylenglycol monomethyl ether, diethylene glycol butyl ether, diethylenglycol monoethylether, diethylenglycol dibutylether, polyethyleneglycol-300, polyethylene glycol-400, propylene glycol, glycerine, 2-pyrrolidone, N-methyl 2-pyrrolidone, glycerol formal, dimethyl sulfoxyde, dibutyl sebecate, polysorbate 80, propylenglycol, and mixtures thereof.

3. A pharmaceutical or veterinary kit according to claim 1 or 2, wherein the solvent of the second container comprises an antioxidant is selected from the group consisting of sodium bisulfite, sodium sulfite, sodium metabisulfite, sodium thiosulfate, L-ascorbic acid, crythorbic acid, acetylcysteine, cysteine, thiourea, monothioglycerol, thioglycolic acid, thiolactic acid, dithiothreitol,dithioerythreitol, glutathione, ascorbyl palmitate, butylated hydroxyanisole, butylated hydroxytoluene, propyl gallate, α-tocopherol, and mixtures thereof.

4. A pharmaceutical or veterinary kit according to any of the preceding claims, wherein the solvent of the second container comprises a preservative selected from the group consisting of benzalkonium chloride, benzethonium chloride, benzoic acid, benzyl alcohol, methylparaben, ethylparaben, propylparaben, butylparaben, sodium benzoate, phenol and mixtures thereof.

5. A pharmaceutical or veterinary kit according to any of the preceding claims, wherein the tulathromycin consists of more than 99% of Tulathromycin A and less than 1% of Tulathromycin B.

6. A pharmaceutical or veterinary kit according to any of the preceding claims, wherein the tulathromycin is present in amorphous form.

7. A pharmaceutical or veterinary kit according to any of claims 1-6 for use in a method of treating a bacterial or protozoal infection in a mammal, wherein the lyophilized composition of tulathromycin is reconstituted with a solvent comprising water and optionally a water-miscible solvent, an antioxidant and/or a preservative, before being administered to a mammal in need of such treatment.

8. Method for the preparation of a lyophilized composition comprising a tulathromycin that consists of more than 98% of Tulathromycin A and less than 2% of Tulathromycon B, in form of an addition salt with citric acid or a mixture of citric acid and hydrochloric acid, comprising the steps of:
a) dissolving the tulathromycin in water with citric acid or a mixture of citric acid and hydrochloric acid,
b) adjusting the pH to a pH of 4.0 to 8.0, and
c) lyophilizing the mixture from step a) or b).

## Patentansprüche

1. Pharmazeutisches oder veterinärmedizinisches Kit zur Verabreichung eines Tulathromycins, das aus mehr als 98 % Tulathromycin A und weniger als 2 % Tulathromycin B besteht, an ein Säugetier,
einen ersten und einen zweiten Behälter umfassend, wobei
der erste Behälter eine lyophilisierte Zusammensetzung eines Tulathromycins, das aus mehr als 98 % Tulathromycin A und weniger als 2 % Tulathromycin B besteht, in Form eines Additionssalzes mit Zitronensäure oder einer Mischung aus Zitronensäure und Salzsäure umfasst, und
der zweite Behälter ein Lösemittel umfasst, das Wasser und optional ein wassermischbares Lösemittel, ein Antioxidans und/oder ein Konservierungsmittel umfasst.

2. Pharmazeutisches oder veterinärmedizinisches Kit nach Anspruch 1, wobei das Lösemittel des zweiten Behälters ein wassermischbares Lösemittel umfasst, ausgewählt aus der Gruppe, bestehend aus Ethanol, Isopropanol, Diethylenglycolmonomethylether, Diethylenglycolbutylether, Diethylenglycolmonoethylether, Diethylenglycoldibutylether, Polyethylenglycol-300, Polyethylenglycol-400, Propylenglycol, Glycerin, 2-Pyrrolidon, N-Methyl-2-pyrrolidon, Glycerinformal, Dimethylsulfoxid, Dibutylsebecat, Polysorbat 80, Propylenglycol und Mischungen derselben.

3. Pharmazeutisches oder veterinärmedizinisches Kit nach Anspruch 1 oder 2, wobei das Lösemittel des zweiten Behälters ein Antioxidans umfasst, ausgewählt aus der Gruppe, bestehend aus Natriumbisulfit, Natriumsulfit, Natriummetabisulfit, Natriumthiosulfat, L-Ascorbinsäure, Krythorbinsäure, Acetylcystein, Cystein, Thioharnstoff, Monothioglycerin, Thioglycolsäure, Thiomilchsäure, Dithiothreitol, Dithioerythreitol, Glutathion, Ascorbylpalmitat, butyliertem Hydroxyanisol, butyliertem Hydroxytoluol, Propylgallat, α-Tocopherol und Mischungen derselben.

4. Pharmazeutisches oder veterinärmedizinisches Kit nach einem der vorhergehenden Ansprüche, wobei das Lösemittel des zweiten Behälters ein Konservierungsmittel umfasst, ausgewählt aus der Gruppe, bestehend aus Benzalkoniumchlorid, Benzethoniumchlorid, Benzoesäure, Benzylalkohol, Methylparaben, Ethylparaben, Propylparaben, Butylparaben, Natriumbenzoat, Phenol und Mischungen derselben.

5. Pharmazeutisches oder veterinärmedizinisches Kit nach einem der vorhergehenden Ansprüche, wobei das Tulathromycin aus mehr als 99 % Tulathromycin A und weniger als 1 % Tulathromycin B besteht.

6. Pharmazeutisches oder veterinärmedizinisches Kit nach einem der vorhergehenden Ansprüche, wobei das Tulathromycin in amorpher Form vorliegt.

7. Pharmazeutisches oder veterinärmedizinisches Kit nach einem der Ansprüche 1 bis 6 zur Verwendung in einem Verfahren zur Behandlung einer bakteriellen oder Protozoen-Infektion in einem Säugetier, wobei die lyophilisierte Zusammensetzung von Tulathromycin mit einem Lösemittel wiederhergestellt wird, das Wasser und optional ein wassermischbares Lösemittel, ein Antioxidans und/oder ein Konservierungsmittel umfasst, bevor sie an ein Säugetier verabreicht wird, das einer solchen Behandlung bedarf.

8. Verfahren zur Herstellung einer lyophilisierten Zusammensetzung, umfassend ein Tulathromycin, das aus mehr als 98 % Tulathromycin A und weniger als 2 % Tulathromycin B besteht, in Form eines Additionssalzes mit Zitronensäure oder einer Mischung aus Zitronensäure und Salzsäure,
umfassend die Schritte:
a) Auflösen des Tulathromycins in Wasser mit Zitronensäure oder einer Mischung aus Zitronensäure und Salzsäure,
b) Einstellen des pH-Werts auf einen pH-Wert von 4,0 bis 8,0 und
c) Lyophilisieren der Mischung aus Schritt a) oder b).

## Revendications

1. Kit pharmaceutique ou vétérinaire permettant l'administration d'une tulathromycine qui est constituée de plus de 98 % de tulathromycine A et de moins de 2 % de tulathromycine B à un mammifère, comprenant un premier et un second récipient,
ledit premier récipient comprenant une composition lyophilisée d'une tulathromycine qui est constituée de plus de 98 % de tulathromycine A et de moins de 2 % de tulathromycine B, sous la forme d'un sel d'addition avec de l'acide citrique ou un mélange d'acide citrique et d'acide chlorhydrique, et
ledit second récipient comprenant un solvant comprenant de l'eau et éventuellement un solvant miscible dans l'eau, un antioxydant et/ou un conservateur.

2. Kit pharmaceutique ou vétérinaire selon la revendication 1, ledit solvant du second récipient comprenant un solvant miscible dans l'eau choisi dans le groupe constitué par l'éthanol, l'isopropanol, l'éther monométhylique de diéthylène glycol, l'éther butylique de diéthylène glycol, l'éther monoéthylique de diéthylène glycol, l'éther dibutylique de diéthylène glycol, le polyéthylène glycol 300, le polyéthylène glycol 400, le propylène glycol, la glycérine, la 2-pyrrolidone, la N-méthyl 2-pyrrolidone, le formal de glycérol, le diméthylsulfoxyde, le sébacate de dibutyle, le polysorbate 80, le propylène glycol et leurs mélanges.

3. Kit pharmaceutique ou vétérinaire selon la revendication 1 ou 2, ledit solvant du second récipient comprenant un antioxydant est choisi dans le groupe constitué par le bisulfite de sodium, le sulfite de sodium, le métabisulfite de sodium, le thiosulfate de sodium, l'acide L-ascorbique, l'acide crythorbique, l'acétylcystéine, la cystéine, la thiourée, le monothioglycérol, l'acide thioglycolique, l'acide thiolactique, le dithiothréitol, le dithioérythréitol, le glutathion, le palmitate d'ascorbyle, l'hydroxyanisole butylé, l'hydroxytoluène butylé, le gallate de propyle, l'α-tocophérol, et leurs mélanges.

4. Kit pharmaceutique ou vétérinaire selon l'une quelconque des revendications précédentes, ledit solvant du second récipient comprenant un conservateur choisi dans le groupe constitué par le chlorure de benzalkonium, le chlorure de benzéthonium, l'acide benzoïque, l'alcool benzylique, le méthylparabène, l'éthylparabène, le propylparabène, le butylparabène, le benzoate de sodium, le phénol et leurs mélanges.

5. Kit pharmaceutique ou vétérinaire selon l'une quelconque des revendications précédentes, ladite tulathromycine étant constituée de plus de 99 % de tulathromycine A et de moins de 1 % de tulathromycine B.

6. Kit pharmaceutique ou vétérinaire selon l'une quelconque des revendications précédentes, ladite tulathromycine étant présente sous forme amorphe.

7. Kit pharmaceutique ou vétérinaire selon l'une quelconque des revendications 1 à 6 destiné à être utilisé dans une méthode de traitement d'une infection bactérienne ou protozoaire chez un mammifère, ladite composition lyophilisée de tulathromycine étant reconstituée avec un solvant comprenant de l'eau et éventuellement un solvant miscible dans l'eau, un antioxydant et/ou un conservateur, avant d'être administrée à un mammifère ayant besoin de ce traitement.

8. Méthode de préparation d'une composition lyophilisée comprenant une tulathromycine qui est constituée de plus de 98 % de tulathromycine A et de moins de 2 % de tulathromycine B, sous la forme d'un sel d'addition avec de l'acide citrique ou un mélange d'acide citrique et d'acide chlorhydrique, comprenant les étapes de :
a) dissolution de la tulathromycine dans de l'eau avec de l'acide citrique ou un mélange d'acide citrique et d'acide chlorhydrique,
b) ajustement du pH à un pH de 4,0 à 8,0, et
c) lyophilisation du mélange à partir de l'étape a) ou b).
